# FASCICULE DE BREVET EUROPEEN

(11) **EP 2 309 920 B1**
(45) Date de publication et mention de la délivrance du brevet: **06.07.2016**
(21) Numéro de dépôt: 09745906.9
(22) Date de dépôt: 28.04.2009
(51) Int. Cl.: A61B 5/00, A61B 5/11

(54) **DISPOSITIF D'ACQUISITION ET DE TRAITEMENT DE DONNÉES PHYSIOLOGIQUES D'UN ANIMAL OU D'UN HUMAIN AU COURS D'UNE ACTIVITÉ PHYSIQUE OU MENTALE**
VORRICHTUNG ZUR ERFASSUNG UND AUFBEREITUNG VON PHYSIOLOGISCHEN DATEN EINES TIERS ODER EINES MENSCHEN IM VERLAUF EINER KÖRPERLICHEN ODER GEISTIGEN TÄTIGKEIT
DEVICE FOR ACQUIRING AND PROCESSING PHYSIOLOGICAL DATA OF AN ANIMAL OR OF A HUMAN IN THE COURSE OF A PHYSICAL OR MENTAL ACTIVITY

(30) Priorité: 28.04.2008 FR 0802383
(43) Date de publication de la demande: 20.04.2011
(73) Titulaire: Université du Sud Toulon-Var, 83957 La Garde Cedex 20 (FR)
(72) Inventeur: GIES, Valentin, 83000 Toulon (FR); HASNI, Fabien, 78370 Plaisir (FR)
(74) Mandataire: Colombo, Michel
(86) Numéro de dépôt international: PCT/FR2009/000495
(87) Numéro de publication internationale: WO 2009/138587

(56) Documents cités:
- WO-A-03/055388
- US-A1- 2004 077 954
- US-A1- 2007 130 893
- US-A1- 2007 276 270

## Description

La présente invention concerne un dispositif d'acquisition et de traitement de données physiologiques d'un individu au sens large, qu'il s'agisse d'un animal ou d'un être humain, au cours d'une activité physique ou mentale.

La première application visée par cette invention est celle du suivi des chevaux de course pendant l'entrainement ou la course, étant entendu que d'autres applications sont envisagées dans le champ de la présente invention moyennant certaines adaptations et variantes du dispositif de base d'acquisition et de traitement de données.

Une autre application visée est celle du suivi des performances physiques de sportifs à l'occasion de la pratique de sports individuels ou collectifs.

Une autre application visée, toujours sans caractère limitatif, est celle du suivi de paramètres physiques, physiologiques, biologiques, d'individus soumis à une pression psychologique ou à un « stress », notamment d'ordre professionnel.

L'invention sera donc décrite principalement dans le cadre de son application au suivi de l'entraînement des chevaux de course, mais sans que cette application n'ait aucun caractère limitatif.

### ETAT DE LA TECHNIQUE :

Dans le domaine de l'entraînement et du suivi des chevaux de course, on connaît de par le WO 01/97606 un dispositif d'entraînement automatique des chevaux. Le but poursuivi par ce dispositif est principalement de permettre l'entraînement des chevaux sans intervention humaine. À cet effet, chaque cheval est placé individuellement dans une installation d'entraînement automatisée, dans laquelle il peut courir, soit sur un tapis roulant, soit dans un espace cloisonné qui se déplace lui-même sur un rail disposé autour d'un champ de course, soit dans un appareil circulaire d'entrainement encore appelé un « marcheur ». Afin de suivre la performance du cheval pendant son entraînement, des électrodes sont placées sur le cheval afin de recueillir différents signaux électriques correspondant à l'activité physiologique du cheval, notamment l'activité du coeur, des poumons et des muscles.

Il est clair que ce dispositif automatisé ne correspond pas à l'entraînement d'un cheval en conditions réelles sur un champ de course et en présence d'un jockey, et a comme unique avantage le fait d'entraîner des chevaux quasiment sans supervision humaine. Mais, comme chaque cheval court dans un espace cloisonné, les conditions réelles de course et leur impact sur la physiologie du cheval et sur sa performance à chaque instant ne peuvent pas être mises en évidence. Ainsi, ce dispositif ne permet pas de surveiller l'apparition d'un défaut dans les mouvements d'un cheval, par exemple lorsqu'il court en ligne droite ou dans un virage, ou en situation de fatigue.

On connait en outre de par le document US 2007/0130893, dans le domaine de l'entraînement et du suivi des chevaux de course, un système plus sophistiqué destine à l'instrumentation des chevaux de course, dans le cadre d'un usage en situation réelle de course ou d'entraînement. Ce système connu est basé sur la fixation de plusieurs capteurs de mouvement repartis sur le corps du cheval, et notamment sur ses pattes ou sur ses sabots. Chaque capteur est localisé sur et est associe à une partie du corps de l'animal. Les différents capteurs répartis sont ensuite reliés par une liaison sans fil par un routeur également placé sur le cheval.

Ce système comporte plusieurs inconvénients.

En effet, il est complexe du fait d'une multitude de composants qui doivent fonctionner ensemble et communiquer entre eux en temps réel. En outre, pour autant qu'il existe en réalité, il serait très difficile à mettre en oeuvre en pratique. Un cheval de course, notamment un pur-sang destiné à la course en ligne, est extrêmement nerveux et craintif, voire caractériel, et il est peu envisageable de le faire patienter pour l'équiper avec les différents composants requis par le système décrit. Par exemple, il parait peut envisageable de poser des capteurs sur les sabots d'un tel cheval, ou de les en retirer. Cette difficulté serait encore accrue s'il s'agissait d'équiper plusieurs chevaux notamment afin de comparer leurs performances lors d'un entraînement ou d'une course.

A supposer néanmoins que cet obstacle soit résolu, ce document pose également le problème important de la qualité et de l'exploitation des signaux captés. Des capteurs posés sur les membres d'un cheval de course vont modifier sa course, de sorte que les signaux captés vont être difficiles à interpréter, contrairement à l'objectif de précision et de reproductibilité qui est recherché.

En outre, un capteur fixé sur la patte d'un cheval, par exemple au moyen d'une guêtre s'il s'agit d'un cheval de saut d'obstacle, est susceptible d'avoir une position qui se dérègle selon au moins deux degrés de liberté, à savoir la hauteur sur la patte, et le degré de rotation par rapport à la fixation initiale. Pour quatre pattes, il sera nécessaire de gérer huit degrés de liberté, ce qui entrainera une accumulation des marges d'erreur de positionnement des capteurs, qui se répercutera négativement sur la précision des calculs. En outre, les traitements numériques pour tenir compte des déplacements parasites des capteurs seront très lourds, ce qui entraînera des impossibilités d'obtenir les résultats recherchés, ou des surcoûts importants. Ces inconvénients seraient encore plus rédhibitoires dans le cas où plusieurs chevaux seraient à suivre simultanément

Par ailleurs, dans d'autres domaines applicatifs, il existe sur le marché des produits spécialement conçus pour le suivi des paramètres physiologiques de sportifs, mais ils comportent eux aussi des inconvénients. En particulier, leurs systèmes d'acquisition n'intègrent pas un cardio-fréquencemètre et un capteur de mouvement précis dans un même et unique boitier de mesure, et ils sont incapables de restituer la courbe complète du signal cardiaque, ainsi que ses paramètres essentiels. En outre, ils ne sont pas utilisables comme des périphériques de matériel informatique portable, puisqu'ils utilisent des protocoles de communication très spécifiques avec leur base de traitement des données.

Enfin, les produits connus ne sont pas adaptés à un usage avec une pluralité de porteurs, comme cela peut être nécessaire par exemple pour suivre de façon simultanée les paramètres physiologiques des membres d'une équipe sportive. De l'état de la technique est également connu de : US2007/276270A1 et WO03/055388A.

### BUTS DE L'INVENTION :

Dans le cas particulier des chevaux de course, un but de l'invention est de proposer un système simple et facile à mettre en oeuvre, et qui soit apte à résoudre les inconvénients des systèmes connus dans l'état de la technique.

Un autre but de l'invention est de proposer un système qui puisse indiquer révolution de certains paramètres physiologiques d'un cheval ou d'un groupe de chevaux, en fonction de leur évolution sur le champ de course, avec notamment pour objectif de distinguer certains disfonctionnements musculaires, articulaires ou autres, en fonction de la fatigue de chaque cheval, et en fonction de sa position sur le champ de course. Dans le cas général, un but de l'invention est par conséquent de proposer un système fiable d'acquisition et de traitement de données représentatives de l'activité physique ou de l'état physiologique d'un individu humain ou animal, ou d'un groupe de tels individus. Ce système doit permettre de délivrer en temps réel d'une part un signal représentatif des mouvements spatiaux de chaque individu, et d'autre part de délivrer un signal représentatif d'une grandeur physiologique de chaque individu, tout en assurant que les signaux de mouvements relatifs à chaque individu soient de grande précision, et soient synchronisés avec les signaux physiologiques respectifs afin de pouvoir, non seulement étudier chaque signal instantané en tant que tel, mais surtout étudier l'évolution de chaque signal de façon synchrone, et par conséquent en corrélation avec l'activité physique de chaque individu. Le but recherché est en effet que le système permette d'indiquer comment tels ou tels paramètres physiologiques de l'individu évoluent en fonction de sa situation, et notamment en fonction de son parcours sur le terrain.

Un autre but de l'invention est de proposer un système permettant de mémoriser les données acquises représentatives de l'activité physique de l'individu humain ou animal, localement et/ou de manière distante de l'individu, afin de pouvoir analyser en temps réel ou en différé les données acquises.

Un autre but de l'invention est de proposer un système permettant de transmettre de façon fiable et rapide les données acquises représentatives de l'activité physique de l'individu sur une distance pouvant au choix être proche, moyenne ou longue.

Un autre but de l'invention est de proposer un système aisément adaptable à une variété de situations, allant du suivi des chevaux de course sur un champ de course, au suivi d'un sportif individuel ou d'une pluralité de sportifs sur un terrain de sport, ou au suivi d'un individu dans des situations professionnelles particulières.

Un autre but de l'invention est de proposer un système qui permettre de suivre simultanément les performances de plusieurs individus, tout en offrant aussi la possibilité de faire varier le niveau de détail du suivi individuel, en temps réel.

Dans le domaine du suivi des chevaux de course ou des animaux, un principe de l'invention consiste (contrairement aux enseignements du document US 2007/0130893 qui prévoit de placer des capteurs au plus près de chaque mouvement à analyser, c'est-à-dire sur les membres du cheval), à centraliser au contraire plusieurs capteurs de mouvement et plusieurs capteurs physiologiques dans un boîtier électronique unique pour chaque cheval, ce boîtier étant destiné à être disposé à un endroit du corps de l'animal distante des membres et de préférence proche du centre de gravité de l'animal, où ledit boîtier unique ne génèrera pas d'impact sur les mouvements de l'animal.

Ainsi, on obtiendra des signaux de mouvement fiables et reproductibles, dont pourront être extraits des informations de qualité quant aux mouvements de l'animal, au moyen d'algorithmes de traitement appropriés appliqués aux grandeurs mesurées par les capteurs du boîtier électronique unique.
Afin d'améliorer encore le résultat des traitements algorithmiques effectués sur les données issues des capteurs, il est utile de procéder à une correction de positionnement des capteurs utilisés, au cas où le positionnement du boîtier de capteurs change légèrement pendant la course ou l'entrainement du cheval.

En effet, le positionnement du boîtier électronique contenant les capteurs de mouvement est forcément entaché d'erreurs dues aux contraintes réelles. Il est donc important de corriger cette erreur avant toute utilisation des données à des fins d'analyse ou de mesure. Le fait que tous les capteurs soient localisés au sein d'un unique boîtier indéformable permet de savoir que l'erreur sera la même pour tous les capteurs, ce qui n'est pas le cas dans les systèmes de mesures basés sur plusieurs boîtiers de mesure non solidaires mécaniquement, voire totalement indépendants comme décrit dans le US 2007/0130893.

Cette erreur de positionnement correspond à un changement de référentiel de base par rapport au positionnement théorique parfait, de sorte qu'il est possible de la corriger en appliquant le changement de base inverse. Cette opération de changement de base est réalisée après avoir calculé la matrice de changement de base par l'analyse des corrélations entre les séries de mesures en provenance des différents capteurs (accéléromètre, gyroscope, ...), les séries de référence étant dé-corrélées.

Dans le cas général, l'invention a pour objet un système d'acquisition et de traitement de données conforme à la revendication 1. Les modes de réalisation de l'invention peuvent aussi comporter les caractéristiques des revendications dépendantes.

De préférence, chaque boîtier électronique individuel regroupe des capteurs de mouvement aptes à délivrer en temps réel un signal représentatif des mouvements spatiaux de l'individu, et au moins un capteur physiologique apte à délivrer un signal représentatif d'au moins une grandeur physiologique de l'individu, ainsi qu'un calculateur recevant en entrée les signaux des capteurs, et délivrant en sortie des signaux synchronisés et numérisés, et une mémoire pour le stockage desdits signaux synchronisés et numérisés.

Les moyens de synchronisation des signaux de mouvement et des signaux physiologiques comportent un calculateur recevant en entrée les signaux des capteurs, et délivrant en sortie des signaux synchronisés, soit à une mémoire locale située dans un récepteur local, soit à une mémoire distante située dans un récepteur distant, par l'intermédiaire de moyens de transmission adaptés.

Grâce à cette structure, le système selon l'invention permet de coupler les données physiologiques avec les mouvements correspondants de l'individu.

En particulier, dans le cas d'un cheval de course, on pourra suivre l'évolution, par exemple, d'un état de fatigue musculaire en fonction de la position réelle du cheval sur un champ de course et de l'effort déjà réalisé, et ainsi détecter les fatigues musculaires, ou l'apparition d'anomalies dans le mouvement, en pouvant notamment séparer les phases de course en ligne droite et en virage.

L'équipement de plusieurs chevaux simultanément avec un boîtier électronique individuel et leur synchronisation avec un dispositif de gestion distant, permettra de disposer pour ainsi dire d'un capteur d'équipe, correspondant à l'ensemble des boîtiers individuels de capteurs, et permettant d'acquérir et de traiter simultanément les paramètres physiologiques de plusieurs chevaux, ou, dans le cas plus général, de plusieurs individus.

De préférence, la communication entre les boîtiers électroniques individuels et le dispositif de gestion distant est bidirectionnelle et permet également d'envoyer à chaque boîtier électronique individuel des informations ou des instructions.

En outre, il peut être prévu que les informations et/ou instructions soient restituées par le boîtier électronique individuel sous forme de messages sonores ou vocaux, mais d'autres modes de restitution sont prévisibles : par mode visuel, par la génération d'impulsions électriques, de vibrations mécaniques, ou autres.

Ces caractéristiques sont particulièrement intéressantes dans le cas du suivi de sports collectifs. L'ensemble des capteurs des joueurs d'une équipe constitue alors une forme de capteur d'équipe virtuel. En effet, les informations de mouvement, de position, ou les informations physiologiques de chaque joueur, arrivent en temps réel sur le dispositif de visualisation et de gestion de l'entraîneur, qui peut alors avoir une vision globale de la situation, et prendre des décisions ou donner des instructions individuelles en conséquence.

De façon avantageuse, le dispositif de gestion distant est pourvu de moyens pour régler en temps réel la bande passante de la communication radio avec chaque boîtier électronique individuel. Ainsi, il sera possible à l'utilisateur du dispositif de gestion distant, typiquement l'entraîneur, de faire des « zooms » ou effets de loupe sur l'activité physique de tel ou tel joueur d'une équipe.

Comme ces effets de loupe demanderont davantage de bande passante instantanée pour la communication entre le dispositif de gestion distant et le boîtier individuel concerné, il sera utile de pouvoir faire varier, à partir du dispositif de gestion distant, la bande passante allouée à la communication avec chaque boitier électronique individuel.

Dans un mode de réalisation envisageable, la communication radio entre les boitiers électroniques individuels et le dispositif de gestion distant se fait a l'aide d'un réseau de communication de téléphonie mobile, notamment de type GSM.

Alternativement, la communication radio entre les boitiers électroniques individuels et le dispositif de gestion distant se fait a l'aide d'un réseau de communication autonome dédié, notamment de type Zigbee.

Lorsqu'un suivi des positions des individus sur le terrain est nécessaire, le système selon l'invention comporte en outre des balises de positionnement réparties autour de la zone d'activité des individus, par rapport auxquelles chaque boîtier électronique détermine sa position instantanée et la transmet au dispositif de gestion distant.

Il peut être prévu que les balises de positionnement comportent une fonction de relais de communication entre les boîtiers électroniques individuels et le dispositif de gestion distant, de façon à assurer la continuité de la communication radio entre les boitiers électroniques individuels et le dispositif de gestion distant, lorsque celui-ci est au-delà de la portée directe de communication des boitiers électroniques individuels.

Dans le cas idéal, les boitiers électroniques individuels et le dispositif de gestion distant sont configurés pour fonctionner comme un réseau de communication radio auto-routeur et auto-adaptatif, apte à optimiser automatiquement le tracé de communication entre les boitiers électroniques individuels et le dispositif de gestion distant, en fonction des déplacements des individus.

Selon la nature de l'individu et de son activité physique, il peut être utile d'exploiter sur place tout ou partie des signaux de mouvement et des signaux physiologiques captés, mais il peut également être utile d'exploiter à distance ces signaux, que ce soit en temps réel ou en temps différé. En conséquence le système selon l'invention comporte des moyens de transmission du signal de mouvement et du signal physiologique vers une unité de stockage locale ou distante de stockage et de traitement des signaux reçus.

Selon l'architecture retenue pour le système dans son contexte applicatif, la transmission des données issues des capteurs vers le récepteur local et/ou le récepteur distant peut se faire de plusieurs manières.

Selon un premier mode de réalisation, les moyens de transmission sont couplés au récepteur local au moyen d'une liaison sans fil à courte portée, typiquement de l'ordre de quelques mètres, et cette liaison sans fil est alors notamment de type bluetooth ou « Zigbee ».

Selon un autre mode de réalisation, les moyens de transmission sont couplés au récepteur distant au moyen d'une liaison sans fil de moyenne portée, typiquement de l'ordre de quelques centaines de mètre, et pour Gela une liaison de type « Zigbee » peut également convenir. Les données transmises peuvent éventuellement être relayées par des routeurs Zigbee, ce qui permet d'étendre la portée du réseau.

Selon un autre mode de réalisation mettant en oeuvre une transmission à plus longue distance entre le récepteur local et un récepteur distant associé à un serveur centralisé, le récepteur local est couplé au récepteur distant au moyen d'une liaison sans fil de longue portée, notamment au moyen d'un réseau de téléphonie mobile sans fil de type GSM ou équivalent.

De façon avantageuse, le calculateur est associé à des moyens d'affichage permettant l'affichage des données issues des capteurs de mouvement et des capteurs physiologiques, et/ou de représentations graphiques de ces données.

Le système selon l'invention permet une grande flexibilité en fonction des activités réelles à analyser. Cette flexibilité est notamment basée sur la variété des capteurs de mouvement et des capteurs physiologiques qui peuvent être mis en oeuvre.

De façon avantageuse, le capteur de mouvement est pris dans un ensemble de capteurs comportant un accéléromètre, un gyroscope, un magnétomètre, ou un capteur de pression.

Alternativement ou en complément, le capteur de mouvement est pris dans un ensemble de balises de positionnement relatif, incluant des balises de type GPS (pour « Global Positioning System » en terminologie anglo-saxonne), des balises à ultrasons, des balises basées sur un réseau à ultra large bande, ou des balises basées sur un protocole de communication de type « Zigbee ».

Selon l'invention, le capteur physiologique est pris dans un ensemble de capteurs comportant les capteurs de signal cardiaque ECG-EKG, les capteurs d'activité musculaire EMG, les capteurs d'activité cérébrale EEG, les capteurs de température corporelle, les capteurs de débit sanguin, et les capteurs d'analyse sanguine embarquée.

Dans le cas du suivi des chevaux de course, le système est de préférence configuré de manière à ce que les capteurs de mouvement et les capteurs physiologiques soient couplés par l'intermédiaire d'une transmission à courte portée, notamment de type « bluetooth » ou « Zigbee », à un récepteur local sous la forme d'un terminal de type assistant numérique personnel ou de type téléphone mobile. Ainsi, le jockey peut suivre en temps réel pendant la course et/ou l'entraînement, certains paramètres physiologiques du cheval, voire adapter l'allure du cheval en conséquence.

En variante, les boitiers de capteurs de mouvement et de capteurs physiologiques sont couplés par une transmission à moyenne portée, de l'ordre de 300 mètres, notamment de type « Zigbee », à un terminal distant, connecté à des moyens d'affichage et de traitement 23, par exemple un ordinateur personnel. Ce terminal de réception distant est notamment utilisé par un entraineur du sportif ou de l'équipe de sportifs suivis, et il possède des moyens de communication bidirectionnelle avec les boîtiers de capteurs individuels, de façon à pouvoir leur transmettre des informations ou des instructions.

En autre variante, les capteurs de mouvement et les capteurs physiologiques sont couplés par une transmission à portée quelconque, notamment de type « GSM », à un serveur distant.

Les capteurs de mouvement utilisés dans le cas du suivi d'un cheval de course comportent par exemple un accéléromètre à trois axes, un capteur GPS pour fournir le tracé du déplacement du cheval, et les capteurs physiologiques comportent un électrocardiographe fournissant le signal cardiaque du cheval en temps réel. En option, les capteurs physiologiques pourront comporter en outre notamment un capteur d'analyse sanguine. Le boitier de capteurs est fixé par une sangle sous le ventre du cheval. En outre, tous les capteurs de mouvement sont localisés au sein d'un unique boitier indéformable de sorte que toute dérive de positionnement dudit boitier entrainera une dérive équivalente pour l'ensemble des capteurs de mouvement, correspondant à un changement de base de référence et apte à être corrigée en appliquant un changement de base inverse.

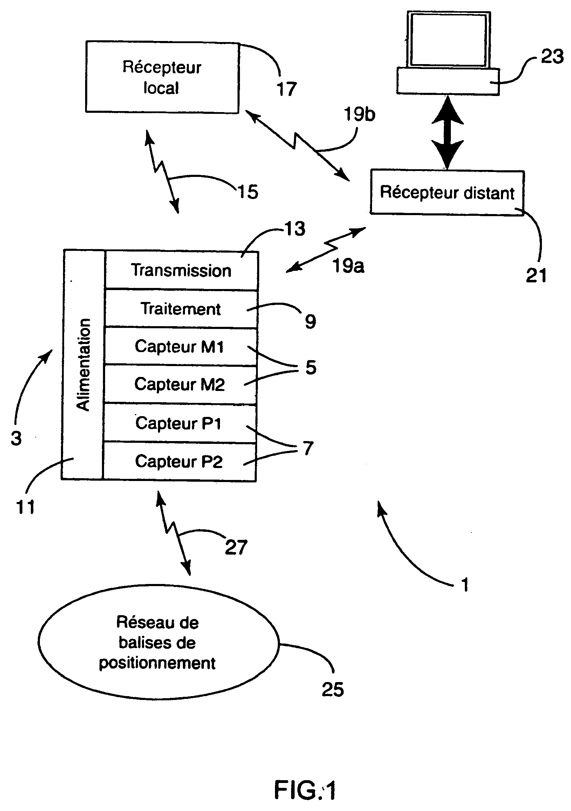

Les caractéristiques et avantages de l'invention apparaîtront à la lecture de la description détaillée des dessins annexés dans lesquels la Figure 1 illustre un organigramme fonctionnel général du système selon l'invention.

On se réfère à la Figure 1. Dans cette figure, on a représenté un organigramme fonctionnel du système 1 selon l'invention.

Ce système 1 comporte en premier lieu, pour chaque individu dont on veut suivre les paramètres de mouvement et certains paramètres physiologiques, un boitier électronique d'acquisition 3 des signaux de mouvement et des signaux physiologiques. Ce boitier 3 comporte au moins un capteur de mouvement 5, et au moins un capteur physiologique 7. Ces capteurs sont regroupés au sein du même boitier, ce qui, combiné à une localisation adéquate du boitier sur l'individu, permet de minimiser les erreurs sur les signaux captés. Ils sont connectés à une unité de prétraitement local 9, soit par une liaison filaire, soit par une liaison sans fil, typiquement à courte portée, auquel cas les capteurs sont autoalimentés. Dans le cas contraire, les capteurs peuvent être alimentés par une alimentation commune 11 faisant partie intégrante du boitier d'acquisition 3, et alimentant également l'unité 9 et une unité de transmission sans fil 13. Les sorties des capteurs sont connectées à l'unité de transmission sans fil 13, qui permet de transmettre à distance les signaux issus des capteurs.

En fonction de la nature des capteurs, qui sera détaillée plus loin, il peut être utile d'effectuer, dans l'unité de traitement 9, un prétraitement local des signaux analogiques ou numériques issus des capteurs 5,7, afin de disposer localement sur le boitier d'acquisition 3 de certains paramètres issus des capteurs. Dans ce cas c'est plutôt la sortie de l'unité de traitement 9 qui est connectée à l'unité de transmission 13, au lieu de la sortie des capteurs 5,7.

L'unité de transmission 13 peut être connectée par une liaison sans fil à courte portée 15, notamment de type « blue tooth » ou « Zigbee », à un récepteur local 17, qui permet notamment de recevoir et d'afficher localement les paramètres de mouvement et les paramètres physiologiques issus des capteurs, ou issus de l'unité de traitement 9 dans le cas où il est utile de disposer d'informations de mouvement et d'informations physiologiques déjà quelque peu synthétisées pour être compréhensibles par l'utilisateur du système.

Ainsi, dans un mode de réalisation avantageux de l'invention, le récepteur local 17 sera constitué par un boitier de communication mobile, de type PDA (« Personal Digital Assistant » en terminologie anglo-saxonne) ou de type téléphone mobile, disposant notamment de capacités d'affichage en temps réel des informations issues des capteurs, et éventuellement de capacités de mémorisation et de traitement. Cette configuration est notamment utile dans le cas du suivi des chevaux de course, puisqu'elle permet au jockey de visualiser en temps réel sur un récepteur local, les paramètres physiologiques de son cheval.

Selon l'invention, il peut également être utile de transmettre à une plus grande distance les données issues des capteurs, afin de les mémoriser et de les traiter plus en détail, que ce soit en temps réel ou en temps différé.

A cet effet, l'invention prévoit de connecter l'unité de transmission 13 du boitier 3 de capteurs, à un récepteur distant 21, au moyen d'une liaison sans fil de moyenne portée 19a.

En variante ou en complément, il est possible selon les besoins de l'application spécifique, de prévoir de connecter le récepteur local 17 audit récepteur distant 21, au moyen d'une liaison sans fil de moyenne ou de longue portée 19b.

Ainsi, dans le cas particulier du suivi des chevaux de course, le jockey pourra disposer d'informations au coeur de l'action, grâce à son récepteur local 17, mais l'écurie pourra avoir les mêmes informations, voire des informations plus complètes, en bordure du champ de course, grâce au récepteur distant 21 connecté par une liaison sans fil soit au boitier 3 de capteurs, soit au récepteur local 17.

Bien entendu, afin d'exploiter de façon complète les données de mouvement et les données physiologiques issues de l'individu qui porte le boitier 3 de capteurs, le récepteur distant 21 est de préférence connecté à une station de traitement et de visualisation 23, qui pourra par exemple être constituée par un ordinateur personnel.

Il est à noter que les grandeurs physiologiques que l'on cherche à capter et à analyser sont directement prélevées par les capteurs physiologiques 7. En ce qui concerne les capteurs de mouvement 5, ils sont également situés uniquement sur le boitier 3 de capteurs qui est autonome dans sa mission d'acquisition des grandeurs de mouvement et des grandeurs physiologiques. Mais il peut également être nécessaire de suivre les mouvements spatiaux de l'individu par référence à un réseau 25 de balises de positionnement absolu, externe par rapport à l'individu, tel que par exemple le réseau des balises de positionnement de type GPS (pour « Global Positioning System »), ou autre. Dans ce cas, l'unité de transmission 13 du boitier 3 de capteurs sera également connectée par une liaison sans fil 27 au réseau de balises de positionnement 25.

On va maintenant décrire plus en détail les boitiers électroniques d'acquisition 3, et les capteurs qu'ils utilisent.

Chaque boitier électronique individuel permet d'effectuer des mesures précises sur un individu. Ces mesures sont les suivantes :

Les mesures de position, qui sont effectuées à l'aide d'un système de positionnement absolu (par exemple de type GPS) ou relatif (par exemple par triangulation de la position par rapport à des balises situées à des emplacements fixes).

Les mesures de mouvements, qui sont effectuées à l'aide d'un accéléromètre 3 axes, et/ou d'un gyroscope 3 axes. Elles permettent de rendre compte des mouvements spatiaux de l'individu qui les porte.

Les mesures cardiaques, qui sont effectuées à l'aide d'un capteur EKG et permettent de capter le signal cardiaque et d'en déduire certains paramètres caractéristiques, comme par exemple le rythme cardiaque, (par exemple pour détecter d'éventuelles anomalies) du sportif à tout instant.

Tous ces capteurs sont connus en eux-mêmes et ne feront par conséquent pas l'objet d'une description détaillée.

Le boitier 3 permet en outre de transmettre les informations par liaison radio sans fil à courte, moyenne ou longue distance (pouvant être supérieure à 1km). Chaque boitier 3 peut être connecté à un récepteur qui lui est propre ou à un récepteur commun. Dans le premier cas (un récepteur par boitier), les données provenant de chacun des boitiers sont ensuite centralisées vers le système informatique de traitement, visualisation et stockage, comme le récepteur distant 21 associé à la station 23. Dans le second cas (un récepteur commun pour tous les boitiers), les données sont transmises à un boitier de réception unique capable d'identifier la provenance des messages reçus et de les transmettre au système informatique de traitement, visualisation et stockage. Ce système informatique de traitement permet de :
- visualiser en temps réel sur un écran de contrôle interactif la position des individus (chevaux, sportifs, etc.) dans leur environnement, et les informations individuelles et collectives les concernant.
- enregistrer les données pour une utilisation ultérieure, en particulier l'analyse à posteriori et le suivi à long terme des individus.
- analyser en temps réel ou à posteriori les informations individuelles ou collectives des individus équipés, en fonction de critères définis préalablement.

L'analyse en temps différé des performances à l'aide du système selon l'invention à déjà été expérimentée par la demanderesse et fonctionne parfaitement dans le domaine équin. Elle peut aisément être adaptée pour améliorer le suivi de l'activité physique d'autres types d'individus et pour d'autres disciplines individuelles ou collectives.

On va maintenant donner des exemples de capteurs de mouvement et de capteurs physiologiques susceptibles d'être utilisés pour mettre en oeuvre l'invention :
Les capteurs présents dans le système peuvent être répartis en deux classes : les capteurs de mouvement, et les capteurs physiologiques.

Parmi les capteurs de mouvement, on distingue :
- **L'accéléromètre :** connu dans l'état de l'art, il permet de connaitre l'accélération dans n directions (avec n =1, 2 ou 3).
- **Le Gyroscope :** connu dans l'état de l'art, il permet de connaitre la vitesse de rotation d'un corps selon n axes de rotation.

L'accéléromètre ne permet pas de déterminer la position et la vitesse qui sont les intégrales de l'accélération. Le gyroscope ne permet pas de déterminer les angles réels qui sont les intégrales des vitesses de rotation. Dans un cas comme dans l'autre, il faut pouvoir déterminer les constantes d'intégration. Pour cela on utilise d'autres capteurs de mouvement, parmi les suivants :
- **Le magnétomètre :** connu dans l'état de l'art, il permet d'obtenir un angle par rapport à la direction d'un champ magnétique (éventuellement terrestre) présent localement.
- **Le capteur de pression :** connu dans l'état de l'art, il permet de mesurer la variation d'altitude ou de profondeur en considérant la pression ambiante constante à une altitude fixée.

On peut également suivre les mouvements spatiaux d'un individu à l'aide d'un système de positionnement par balises, parmi lesquelles on pourra prévoir plusieurs possibilités :
- **Les balises GPS :** connues dans l'état de l'art, elles permettent d'obtenir la position du capteur (et donc sa vitesse par dérivation) par rapport au référentiel terrestre. Elles sont relativement imprécises {précision de 10m environ en mode normal et 2m en mode différentiel) et elles ont une fréquence d'acquisition faible (1-5Hz).
- **Les balises à ultrasons :** elles sont connues dans l'état de l'art, permettent un positionnement précis (1cm) dans un environnement de taille réduite (quelques centaines de mètres) où sont placés des émetteurs à ultrasons.
- **Les balises à ultra large bande, ou « Ultra Wide Band » en terminologie anglo-saxonne :** elles sont connues dans l'état de l'art, et permettent un positionnement assez précis (10cm) dans un environnement de taille réduite (quelques centaines de mètres) où sont placés des émetteurs.
- **Les balises dites « Zigbee** » : elles sont peu connues dans l'état de l'art existant, elles permettent un positionnement peu précis mais utilisant comme balise un réseau de communication utilisant un protocole de communication « Zigbee » utilisé aussi pour la transmission des données.

Les systèmes de balises permettent de connaitre (avec une précision dépendant du système choisi) la position avec une fréquence dépendant du système choisi mais relativement faible (de l'ordre de quelques dizaines de Hz au maximum).

Afin d'améliorer encore le suivi des mouvements, l'invention prévoit de coupler certains capteurs de mouvement au sein du boitier d'acquisition 3. On retiendra :
**- Le couplage d'un accéléromètre et d'un système de position par balises :** il permet d'obtenir des informations précises sur la position avec une fréquence d'échantillonnage élevée (1kHz ou plus). Les données accélérométriques sont intégrées une ou deux fois (pour obtenir la vitesse ou la position), et les dérives dues à un décalage sur le capteur ou à une mauvaise constante d'intégration sont corrigées grâce au système de positionnement (qui donne une position ou une vitesse absolue). On l'appellera dans la suite un **accéléromètre compensé.**
**- Le couplage d'un accéléromètre et d'un capteur de pression :** il permet d'obtenir des informations précises sur l'altitude ou la profondeur avec une fréquence d'échantillonnage élevée (1kHz ou plus). Les données accélérométriques sont intégrées deux fois, et les dérives sont corrigées grâce au capteur de pression (qui donne l'altitude absolue). On l'appellera dans.la suite un **altimètre compensé.**
**- Le couplage d'un gyroscope et d'un magnétomètre :** il permet d'obtenir des informations très précises (<1° d'erreur) et haute fréquence (1KHz) sur l'angle du capteur, le principe est le même que précédemment : les données du gyroscope sont intégrées une fois, et la dérive est corrigées à l'aide du magnétomètre. On l'appellera dans la suite un **gyroscope compensé.**

Selon l'invention, on associe à l'un des capteurs de mouvement décrits précédemment, un capteur physiologique, de manière à pouvoir associer précisément les grandeurs représentatives du mouvement spatial de l'individu, à certaines des grandeurs décrivant son activité physiologique correspondante.

Parmi les capteurs physiologiques, on retiendra :
- **L'électrocardiographe** (ECG ou EKG): connu dans l'état de l'art, il permet d'obtenir le signal ECG lié aux battements du coeur. Il peut avoir plusieurs voies, et chaque voie utilise une électrode, à laquelle il faut ajouter une électrode de masse commune pour toutes les voies. L'acquisition est effectuée à fréquence élevée (>50Hz) de manière à pouvoir observer finement le signal.
- **L'électromyographe** (EMG) : connu dans l'état de l'art, il permet d'analyser l'activité électrique des musdes. Les acquisitions se font également à fréquence élevée (> 500 Hz).
- **L'électroencéphalographe** (EEG) : connu dans l'état de l'art, il permet d'analyser l'activité électrique du cerveau. Les acquisitions se font également à fréquence élevée (> 500 Hz).
- **Les capteurs de température :** bien connus dans l'état de l'art, ils permettent de mesurer la température. Il est prévu d'en utiliser plusieurs exemplaires pour mesurer la température en différents points du corps de l'individu durant un test. L'architecture générale du système 1 d'acquisition et de traitement telle qu'elle vient d'être décrite peut convenir au suivi de l'activité d'individus dans un grand nombre de situations applicatives, moyennant quelques adaptations à la portée de l'homme du métier en fonction de chaque application spécifique.

Certaines applications spécifiques et les adaptations correspondantes du système 1 vont maintenant être décrites.

Ainsi, les capteurs de mouvement et les capteurs physiologiques décrits précédemment peuvent être couplés entre eux avec à propos, en fonction du type de test réalisé, dont plusieurs exemples vont maintenant être donnés.

**Le test de locométrie :** ce type de test vise à déterminer les caractéristiques mécaniques d'individus au cours d'un effort en situation réelle. Ce test est particulièrement utile, dans le cadre de l'invention, au suivi et à l'analyse des paramètres de mouvement et des paramètres physiologiques dans le cas des chevaux de course. Il requiert l'usage d'un capteur d'équipe composé d'un ou plusieurs capteurs de mouvement compensés ou non. Les capteurs communiquent avec un terminal informatique de visualisation et enregistrement de données permettant d'analyser en temps réel ou différé et de manière comparative les performances entre plusieurs chevaux qui sont en cours de test ou dont les performances ont été déjà enregistrées au préalable. Le capteur d'équipe permet en outre au superviseur de se focaliser sur un individu en particulier de manière à affiner le diagnostic.

Les capteurs de mouvement permettent d'enregistrer à fréquence élevée les données relatives à ce mouvement de manière à mettre en évidence à l'aide de méthodes et d'algorithmes de traitement du signal (non décrits ici) les caractéristiques du mouvement, et éventuellement les problèmes qui y sont liés.

Dans le cas de l'application au cheval, chaque capteur de mouvement comporte un accéléromètre à trois axes, un capteur GPS fournissant le tracé du déplacement du cheval, et un électrocardiographe fournissant le signal cardiaque du cheval en temps réel. L'utilisation d'un capteur physiologique de type EMG couplé au précédent permet de détecter en plus des caractéristiques et problèmes mécaniques du sujet ses caractéristiques et problèmes musculaires.

En outre, l'usage de capteurs de mouvement compensés peut être d'un grand intérêt pour affiner les mesures, il permet en effet de connaître plus précisément le déplacement à une échelle de temps de plusieurs secondes. Cette information permet de corréler les mesures à haute fréquence caractéristiques du fonctionnement locomoteur du sujet au déplacement global. Cela permet de mettre en évidence des phénomènes tels qu'un problème locomoteur lors d'une phase où le sujet se déplace selon un mouvement caractéristique (par exemple en courbe, en montée ou en descente...).

### Les tests d'effort, d'endurance ou de déplacement :

Ce type de test vise à déterminer le potentiel d'un individu dans le cadre d'un test d'effort déterminé.

On utilise en général un capteur de mouvement utilisant un accéléromètre compensé et éventuellement un gyroscope (éventuellement compensé), couplé à des capteurs physiologiques. Outre les « micro » données loco-métriques enregistrées à haute fréquence et décrites précédemment, l'intérêt de ces tests est de générer des « macro » données plus synthétiques et à fréquence plus réduite (typiquement 1 à 5 Hz, ce qui limite la bande passante nécessaire pour la transmission sans fil) permettant de caractériser l'évolution de certains paramètres physiologiques et loco métriques au cours d'un essai. Ainsi, à titre d'exemple, dans le domaine des signaux cardiaques, une micro-donnée sera constituée par l'intégralité du signal cardiaque en fonction du temps, et une macro-donnée issue de la micro-donnée pourra être constituée par le rythme cardiaque en pulsations par minute.

Les capteurs utilisés sont ceux des tests loco-métriques, à la différence près qu'il est indispensable d'utiliser des capteurs compensés de manière à connaitre des données telles que la vitesse ou le parcours effectué par le sujet et non plus seulement son accélération ou sa vitesse de rotation instantanée. On peut ajouter d'autres capteurs: un ou plusieurs capteurs de température permettant d'obtenir à intervalles réguliers (avec une fréquence de 1 Hz environ) des informations complémentaires sur la température corporelle du sujet dans des conditions de température extérieure particulières, un électroencéphalogramme EEG permettant d'enregistrer l'évolution de l'activité cérébrale au cours d'un test (par exemple un test d'endurance).

La mise en correspondance, notamment par l'unité de traitement 9, des « macro » données ECG, EMG, EEG et des données décrivant le mouvement de l'individu permet d'obtenir des informations couplées particulièrement pertinentes telles que : activité musculaire et cardiaque correspondant à un effort ponctuel donné pendant un test d'effort, temps de récupération après l'effort (test de récupération), évolution du rythme cardiaque, de l'activité musculaire, cérébrale et des caractéristiques du déplacement au cours d'un essai de longue durée {test d'endurance) ou dans le cadre d'une activité impliquant plusieurs sujets sport collectif...).

**Les tests physiologiques avancés, comme** la **mesure du stress :**
Ce type de mesure vise à caractériser le comportement d'un sujet en situation de stress. Il requiert l'usage d'un capteur de type ECG ou/et EEG éventuellement couplé à un capteur de mouvement (non compensé). Cet ensemble permet d'observer l'évolution du rythme cardiaque et des mouvements dus à la nervosité et au stress lorsqu'un sujet est soumis à un test de stress. A cet effet, le système selon l'invention est adapté et comporte au moins un capteur de mouvement pris parmi un accéléromètre à trois axes, un magnétomètre, un capteur de pression, un gyroscope, et un capteur de position de type GPS, un capteur à ultrasons de type Zigbee ou à ultra large bande, et au moins un capteur physiologique pris parmi un électromyographe, au moins un capteur de température, un électrocardiographe ECG, un électroencéphalographe EEG. De préférence, chacun desdits capteurs a au moins une voie.

Les tests physiologiques ainsi conduits à l'aide des boitiers de capteurs précités sont particulièrement adaptés à la gestion du stress dans un ensemble de professions à risques, comme notamment les pompiers ou les policiers.

**Le suivi de sportifs évoluant dans un espace délimité tel qu'une salle ou un terrain de sports :**
Le système selon l'invention peut également être adapté pour ce type d'application.

Le système est utilisé en tant que capteur d'équipe au sens ou le superviseur (entraineur ou autre) peut visualiser, enregistrer et rejouer sur un terminal informatique les données relatives à ses joueurs : position sur le terrain, état de fatigue, distance parcourue, caractéristiques physiques à l'instant de la mesure, historique des caractéristiques physiques par exemple.

Le superviseur peut également choisir à tout instant d'affiner la mesure sur un individu particulier de manière à pouvoir effectuer une analyse fine, voir un diagnostic médical en temps réel sur celui-ci. Dans ce cas, le capteur d'équipe adapte automatiquement ses flux de données de manière à laisser plus d'information arriver en provenance de l'individu concerné.

Les capteurs de mouvement comportent au moins trois balises à ultrasons couplées à un dispositif de synchronisation radio, et chaque joueur est pourvu d'un capteur à ultrasons permettant de mesurer au choix : les distances entre chaque balise et le capteur (le capteur nécessite alors une synchronisation radio avec les émetteurs) ou les différences entre ces distances (le capteur ne nécessite pas dans ce cas de synchronisation avec les émetteurs mais les traitements à effectuer sont plus complexes), de façon à déterminer la position relative de chaque joueur par rapport à l'ensemble des émetteurs avec une fréquence de l'ordre de 5 à 10 Hz.

Dans le cas où les sportifs évoluent à l'extérieur, le système doit être adapté et les capteurs de mouvement comportent des balises de type GPS, éventuellement couplées à des balises à ultrasons, des balises « Zigbee » ou des balises à ultra large bande.

Dans les deux cas, les capteurs physiologiques comportent un cardio-fréquencemètre dont le signal est traité de manière à obtenir le rythme cardiaque de chaque joueur.

**La fourniture de données à des applications de type Web distantes :**
Le système selon l'invention peut également être adapté pour ce type d'application. Dans ce cas, les données en provenance du capteur d'équipe constitué d'un ensemble de boitiers d'acquisitions et d'une unité de supervision et d'enregistrement sont transmises à un serveur distant en utilisant une liaison de type GSM (téléphone portable). Puis elles sont stockées dans une base de données de manière à être utilisées par des applications Web spécifiques, comme par exemple les réseaux sociaux sur internet, ou équivalent. Le serveur web distant traite les données selon des algorithmes applicatifs spécifiques, qui ne sont pas décrits ici, mais qui sont à la portée de l'homme du métier.

### AVANTAGES DE L'INVENTION :

L'intérêt du système selon l'invention est de fournir une plateforme de capteurs flexible et aisément adaptable à plusieurs types d'applications, comme le suivi des performances des chevaux de course, ou le suivi d'activité humaines sportives ou non. Le système intègre notamment dans un seul boitier électronique un capteur de mouvement et un cardio-fréquencemètre, et pourra intégrer à terme plusieurs capteurs supplémentaires si besoin.

Dans certaines applications, comme le suivi des paramètres de mouvement des chevaux de course, la fixation et la localisation du boitier électronique unique sous le ventre du cheval, tel que prévu par l'invention, est particulièrement importante pour le résultat final, et permet de simplifier considérablement le traitement des signaux, notamment ceux liés aux mouvements du cheval, afin d'en extraire des informations sur l'existence et la localisation de tel ou tel problème, par exemple au niveau d'une articulation d'une patte.

Le boitier de capteurs fonctionne comme un périphérique de téléphone portable, de PDA ou de terminal informatique standard, ce qui évite l'achat d'un récepteur dédié.

L'utilisation du système permet de fournir et d'analyser des données de mouvement et des données physiologiques correspondantes, avec un niveau de précision et de détail bien plus grand que par le passé, ce qui permet notamment une optimisation de l'entrainement des chevaux de course, ou des sportifs pratiquant un sport collectif ou individuel.

En particulier, dans le cas de chevaux de course, on pourra suivre l'évolution, par exemple, d'un état de fatigue musculaire en fonction de la position réelle du cheval sur un champ de course et de l'effort déjà réalisé, en comparaison avec d'autres chevaux effectuant un effort similaire simultanément, et ainsi détecter les fatigues musculaires, ou l'apparition d'anomalies dans le mouvement, en pouvant notamment séparer les phases de course en ligne droite et en virage.

L'acquisition des données s'effectue sans fil sur un terminal de type téléphone portable, PDA, tablet PC ou ordinateur équipé d'une liaison radio (par exemple Bluetooth ou Zigbee) de manière à ne pas gêner les mouvements de l'animal ou de l'individu.

Les atouts du système sont principalement la récupération d'informations précises et complètes sur les performances des individus (biomécanique, cardiologie et déplacements), la visualisation, la comparaison et l'analyse en temps réel de l'évolution de ces informations de manière individuelle et collective, que ce

L'acquisition des données s'effectue sans fil sur un terminal de type téléphone portable, PDA, tablet PC ou ordinateur équipé d'une liaison radio (par exemple Bluetooth ou Zigbee) de manière à ne pas gêner les mouvements de l'animal ou de l'individu.

Les atouts du système sont principalement la récupération d'informations précises et complètes sur les performances des individus (biomécanique, cardiologie et déplacements), la visualisation, la comparaison et l'analyse en temps réel de l'évolution de ces informations de manière individuelle et collective, que ce soit localement ou à distance, et la possibilité d'un suivi à long terme des individus, permettant d'optimiser leurs performances collectives et individuelles.

La possibilité de régler automatiquement et de façon dynamique entre un boitier de capteurs et le dispositif de gestion distant, permet de faire un zoom sur l'activité physique d'un individu dans une équipe.

L'invention permet aussi de réaliser une expertise technique avancée des performances biomécaniques et physiologiques d'humains ou d'animaux.

## Revendications

1. Système d'acquisition et de traitement de données représentatives de l'activité physique ou mentale et/ou de l'état physiologique d'une pluralité d'individus humains ou animaux, dans lequel il comporte :
a) pour chaque individu, un boîtier électronique individuel et unique au sein duquel sont regroupés plusieurs capteurs susceptibles de mesurer des grandeurs physiques et/ou biologiques liées à l'activité physique du porteur du boîtier,
b) chaque boîtier électronique étant en outre pourvu de moyens de communication radio avec un dispositif de gestion distant permettant la gestion des données recueillies à partir des boîtiers individuels,
et en ce que :
- chaque boîtier électronique individuel est apte à transmettre un message contenant les données mesurées par ses capteurs vers l'un, unique, des boîtiers électroniques individuels, appelé « boîtier de réception », et
- le boîtier de réception est apte à identifier la provenance des messages reçus et à les transmettre au dispositif de gestion distant.

2. Système selon la revendication 1, **caractérisé en ce que** chaque boîtier électronique individuel regroupe des capteurs de mouvement (5) aptes à délivrer en temps réel un signal représentatif des mouvements spatiaux de l'individu, etau moins un capteur physiologique (7) apte à délivrer un signal représentatif d'au moins une grandeur physiologique de l'individu, ainsi qu'un calculateur recevant en entrée les signaux des capteurs, et délivrant en sortie des signaux synchronisés et numérisés, et une mémoire pour le stockage desdits signaux synchronisés et numérisés.

3. Système selon la revendication 1 ou la revendication 2, **caractérisé en ce que** la communication entre les boîtiers électroniques individuels et le dispositif de gestion distant est bidirectionnelle et permet également d'envoyer à chaque boîtier électronique individuel des informations ou des instructions.

4. Système selon la revendication 3, **caractérisé en ce que** les informations et/ou instructions reçues sont restitués par le boîtier électronique individuel sous la forme de messages vocaux, de signaux lumineux, sonores ou électriques.

5. Système selon la revendication 3, **caractérisé en ce que** le dispositif de gestion distant est pourvu de moyens pour régler en temps réel la bande passante de la communication radio avec chaque boîtier électronique individuel.

6. Système (1) selon l'une quelconque des revendications 1 à 5, **caractérisé en ce qu'**il est optimisé pour le suivi de l'activité physique d'un ou de plusieurs chevaux sur un champ de course ou une aire de compétition équestre, et **en ce qu'**il comporte, pour chaque cheval, un boîtier électronique individuel et unique, fixé à l'écart des membres et au voisinage de son centre de gravité.

7. Système (1) selon la revendication 6, **caractérisé en ce que** tous les capteurs de mouvement sont localisés au sein d'un unique boîtier (3) indéformable de sorte que toute dérive de positionnement dudit boîtier entraînera une dérive équivalente pour l'ensemble des capteurs de mouvement, correspondant à un changement de base de référence et apte à être corrigée en appliquant un changement de base inverse.

8. Système selon la revendication 6 ou 7, **caractérisé en ce que** le boîtier électronique individuel est fixé par une sangle sous le ventre du cheval.

9. Système selon les revendications 2 et 6 prises en combinaison, **caractérisé en ce que** les capteurs de mouvement (5) comportent un accéléromètre à trois axes, un capteur GPS fournissant le tracé du déplacement du cheval, et **en ce que** les capteurs physiologiques (7) comportent un électrocardiographe fournissant le signal cardiaque du cheval en temps réel.

10. Système selon la revendication 6, **caractérisé en ce que** les capteurs physiologiques (7) comportent en outre un capteur d'analyse sanguine.

11. Système (1) selon l'une quelconque des revendications 1 à 5, optimisé pour le suivi de sportifs évoluant dans un lieu délimité tel qu'un terrain ou une salle de sports, **caractérisé en ce que** les capteurs de mouvement (5) disposés dans le boîtier électronique individuel comportent au moins trois balises à ultrasons couplées à un dispositif de synchronisation radio, et **en ce que** chaque joueur est pourvu d'un capteur à ultrasons permettant de mesurer au choix : les distances entre chaque balise et le capteur ou les différences entre ces distances, de façon à déterminer la position relative de chaque joueur par rapport à l'ensemble des émetteurs avec une fréquence de l'ordre de cinq à 10 Hz.

12. Système (1) selon l'une quelconque des revendications 1 à 5, optimisé pour le suivi de sportifs évoluant à l'extérieur, **caractérisé en ce que** les capteurs de mouvement (5) disposés dans le boîtier électronique individuel comportent des balises de type GPS, éventuellement couplées à des balises à ultrasons, des balises « Zigbee » ou des balises à ultra large bande.

13. Système (1) selon l'une des revendications 11 à 12, **caractérisé en ce que** les capteurs physiologiques (7) disposés dans le boîtier électronique individuel comportent un cardio-fréquencemètre dont le signal est traité de manière à obtenir le rythme cardiaque de chaque individu.

14. Système (1) selon l'une quelconque des revendications 1 a 5, optimisé pour la détection du stress d'un individu en situation professionnelle, **caractérisé en ce que** les capteurs de mouvement (5) disposés dans le boîtier électronique individuel comportent un accéléromètre et **en ce que** les capteurs physiologiques (7) comportent un cardio-fréquencemètre et un électroencéphalographe.

15. Système (1) selon l'une quelconque des revendications 1 a 5, optimisé pour des tests physiologiques avancés sur des individus, **caractérisé en ce que** les capteurs de mouvement (5) disposés dans le boîtier électronique individuel comportent un ou plusieurs capteurs pris parmi un accéléromètre à trois axes, un magnétomètre, un capteur de pression, un gyroscope, et un capteur de position de type GPS, un capteur à ultrasons de type Zigbee ou à ultra-large bande, et **en ce que** les capteurs physiologiques (7) comportent un ou plusieurs capteurs pris parmi un électromyographe, au moins un capteur de température, un électrocardiographe ECG, un électroencéphalographe EEG, chacun desdits capteurs (5,7) ayant au moins une voie.

## Patentansprüche

1. System zur Erfassung und Verarbeitung von Daten, die für die körperliche oder geistige Aktivität und/oder den physiologischen Zustand einer Vielzahl von menschlichen oder tierischen Individuen repräsentativ sind, das aufweist:
a) für jedes Individuum ein individuelles und einziges Elektronikgehäuse, in dem mehrere Sensoren zusammengefasst sind, die geeignet sind, physikalische und/oder biologische Größen zu messen, die mit der körperlichen Aktivität des Trägers des Gehäuses verbunden sind,
b) wobei jedes Elektronikgehäuse außerdem mit Funkverbindungseinrichtungen zu einer fernen Verwaltungsvorrichtung ausgestattet ist, die die Verwaltung der ausgehend von den individuellen Gehäusen aufgefangenen Daten ermöglicht, und:
- jedes individuelle Elektronikgehäuse fähig ist, eine die von seinen Sensoren gemessenen Daten enthaltende Mitteilung an ein einziges der individuellen Elektronikgehäuse, « Empfangsgehäuse » genannt, zu übertragen, und
- das Empfangsgerät fähig ist, die Herkunft der empfangenen Mitteilungen zu erkennen und sie an die ferne Verwaltungsvorrichtung zu übertragen.

2. System nach Anspruch 1, **dadurch gekennzeichnet, dass** jedes individuelle Elektronikgehäuse Bewegungssensoren (5), die fähig sind, in Echtzeit ein Signal zu liefern, das für die räumlichen Bewegungen des Individuums repräsentativ ist, und mindestens einen physiologischen Sensor (7), der fähig ist, ein für mindestens eine physiologische Größe des Individuums repräsentatives Signal zu liefern, sowie einen Rechner, der am Eingang die Signale der Sensoren empfängt und am Ausgang synchronisierte und digitalisierte Signale liefert, und einen Speicher zum Speichern der synchronisierten und digitalisierten Signale umfasst.

3. System nach Anspruch 1 oder Anspruch 2, **dadurch gekennzeichnet, dass** die Verbindung zwischen den individuellen Elektronikgehäusen und der fernen Verwaltungsvorrichtung bidirektional ist und es ebenfalls ermöglicht, an jedes individuelle Elektronikgehäuse Informationen oder Anweisungen zu schicken.

4. System nach Anspruch 3, **dadurch gekennzeichnet, dass** die empfangenen Informationen und/oder Anweisungen vom individuellen Elektronikgehäuse in Form von Voice-Mails, Licht-, Ton- oder elektrischen Signalen wiedergegeben werden.

5. System nach Anspruch 3, **dadurch gekennzeichnet, dass** die ferne Verwaltungsvorrichtung mit Einrichtungen versehen ist, um die Bandbreite der Funkverbindung mit jedem individuellen Elektronikgehäuse in Echtzeit zu regeln.

6. System (1) nach einem der Ansprüche 1 bis 5, **dadurch gekennzeichnet, dass** es für die Verfolgung der körperlichen Aktivität eines oder mehrerer Pferde auf einer Rennbahn oder einem Reitturnier-Platz optimiert ist, und dass es für jedes Pferd ein individuelles und einziges Elektronikgehäuse aufweist, das von den Gliedmaßen entfernt und in der Nähe seines Schwerpunkts befestigt ist.

7. System (1) nach Anspruch 6, **dadurch gekennzeichnet, dass** alle Bewegungssensoren sich in einem einzigen unverformbaren Gehäuse (3) befinden, so dass jede Positionierabweichung des Gehäuses eine äquivalente Abweichung für die Gesamtheit der Bewegungssensoren nach sich zieht, was eine Bezugsbasisänderung entspricht und korrigiert werden kann, indem eine umgekehrte Basisänderung angewendet wird.

8. System nach Anspruch 6 oder 7, **dadurch gekennzeichnet, dass** das individuelle Elektronikgehäuse durch einen Gurt unter dem Bauch des Pferds befestigt wird.

9. System nach den Ansprüche 2 und 6 in Kombination, **dadurch gekennzeichnet, dass** die Bewegungssensoren (5) einen dreiachsigen Beschleunigungsmesser aufweisen, wobei ein GPS-Sensor den Verlauf der Bewegung des Pferds liefert, und dass die physiologischen Sensoren (7) einen Elektrokardiographen aufweisen, der das Herzsignal des Pferds in Echtzeit liefert.

10. System nach Anspruch 6, **dadurch gekennzeichnet, dass** die physiologischen Sensoren (7) außerdem einen Blutanalysesensor aufweisen.

11. System (1) nach einem der Ansprüche 1 bis 5, optimiert für die Verfolgung von sich in einer begrenzten Umgebung wie einem Sportplatz oder einer Sporthalle bewegenden Sportlern, **dadurch gekennzeichnet, dass** die im individuellen Elektronikgehäuse angeordneten Bewegungssensoren (5) mindestens drei Ultraschallbaken aufweisen, die mit einer Funksynchronisationsvorrichtung gekoppelt sind, und dass jeder Spieler mit einem Ultraschallsensor ausgestattet ist, der es ermöglicht, wahlweise zu messen: die Abstände zwischen jeder Bake und dem Sensor oder die Unterschiede zwischen diesen Abständen, um die relative Stellung jedes Spielers bezüglich der Gesamtheit der Emitter mit einer Frequenz in der Größenordnung von fünf bis 10 Hz zu bestimmen.

12. System (1) nach einem der Ansprüche 1 bis 5, optimiert für die Verfolgung von sich draußen bewegenden Sportlern, **dadurch gekennzeichnet, dass** die im individuellen Elektronikgehäuse angeordneten Bewegungssensoren (5) Baken von der Art GPS aufweisen, die ggf. mit Ultraschallbaken, « Zigbee »-Baken oder Ultrabreitband-Baken gekoppelt sind.

13. System (1) nach einem der Ansprüche 11 bis 12, **dadurch gekennzeichnet, dass** die im individuellen Elektronikgehäuse angeordneten physiologischen Sensoren (7) einen Herzfrequenzmesser aufweisen, dessen Signal so verarbeitet wird, dass der Herzrhythmus jedes Individuums erhalten wird.

14. System (1) nach einem der Ansprüche 1 bis 5, optimiert für die Erfassung der Stressbelastung eines Individuums in einer beruflichen Situation, **dadurch gekennzeichnet, dass** die im individuellen Elektronikgehäuse angeordneten Bewegungssensoren (5) einen Beschleunigungsmesser aufweisen, und dass die physiologischen Sensoren (7) einen Herzfrequenzmesser und einen Elektroenzephalographen aufweisen.

15. System (1) nach einem der Ansprüche 1 bis 5, optimiert für fortgeschrittene physiologische Tests an Individuen, **dadurch gekennzeichnet, dass** die im individuellen Elektronikgehäuse angeordneten Bewegungssensoren (5) einen oder mehrere Sensoren aufweisen, die ausgewählt werden unter einem dreiachsigem Beschleunigungsmesser, einem Magnetometer, einem Drucksensor, einem Gyroskop, und einem Positionsgeber von der Art GPS, einem Ultraschallsensor von der Art Zigbee oder mit Ultrabreitband, und dass die physiologischen Sensoren (7) einen oder mehrere Sensoren aufweisen, die ausgewählt werden unter einem Elektromyographen, mindestens einem Temperaturfühler, einem Elektrokardiographen ECG, einem Elektroenzephalographen EEG, wobei jeder der Sensoren (5, 7) mindestens einen Kanal hat.

## Claims

1. System for acquiring and processing data representative of the physical or mental activity and/or of the physiological state of a plurality of human individuals or animals, in which it comprises:
a) for each individual, an individual and unique electronic case in which are grouped together a number of sensors capable of measuring physical and/or biological quantities linked to the physical activity of the wearer of the case,
b) each electronic case being further provided with radio communication means with a remote management device allowing for the management of the data collected from the individual cases, and:
- each individual electronic case is capable of transmitting a message containing the data measured by its sensors to a single one of the individual electronic cases, called "reception case", and
- the reception case is capable of identifying the origin of the messages received and of transmitting them to the remote management device.

2. System according to Claim 1, **characterized in that** each individual electronic case groups together motion sensors (5) capable of delivering in real time a signal representative of the spatial movements of the individual, and at least one physiological sensor (7) capable of delivering a signal representative of at least one physiological quantity of the individual, and a computer receiving as input the signals from the sensors, and delivering as output synchronized and digitized signals, and a memory for the storage of said synchronized and digitized signals.

3. System according to Claim 1 or Claim 2, **characterized in that** the communication between the individual electronic cases and the remote management device is 2-way and also makes it possible to send information or instructions to each individual electronic case.

4. System according to Claim 3, **characterized in that** the information and/or instructions received are played back by the individual electronic case in the form of voice messages, light, sound or electrical signals.

5. System according to Claim 3, **characterized in that** the remote management devices is provided with means for setting in real time the bandwidth of the radio communication with each individual electronic case.

6. System (1) according to any one of Claims 1 to 5, **characterized in that** it is optimized to track the physical activity of one or more horses on a race track or an equestrian competition area, and **in that** it comprises, for each horse, an individual and unique electronic case, fixed away from the limbs and in the vicinity of its centre of gravity.

7. System (1) according to Claim 6, **characterized in that** all the motion sensors are located within a single non-deformable case (3) such that any drift in positioning of said case will result in an equivalent drift for all the motion sensors, corresponding to a change of reference base and capable of being corrected by applying a reverse change of base.

8. System according to Claim 6 or 7, **characterized in that** the individual electronic case is fixed by a strap under the belly of the horse.

9. System according to Claims 2 and 6 taken in combination, **characterized in that** the motion sensors (5) comprise a tri-axial accelerometer, a GPS sensor providing the plot of the movement of the horse, and **in that** the physiological sensors (7) comprise an electrocardiograph supplying the cardiac signal of the horse in real time.

10. System according to Claim 6, **characterized in that** the physiological sensors (7) further comprise a blood analysis sensor.

11. System (1) according to any one of Claims 1 to 5, optimized to track sports persons moving in a delimited location such as a sports field or hall, **characterized in that** the motion sensors (5) arranged in the individual electronic case comprise at least three ultrasound beacons coupled to a radio synchronisation device, and **in that** each player is provided with an ultrasound sensor making it possible to measure, by choice: the distances between each beacon and the sensor or the differences between these distances, so as to determine the relative position of each player relative to all the emitters with a frequency of the order of five to 10 Hz.

12. System (1) according to any one of Claims 1 to 5, optimized to track sports persons moving outside, **characterized in that** the motion sensors (5) arranged in the individual electronic case comprise beacons of GPS type, possibly coupled to ultrasound beacons, "Zigbee" beacons or ultra-wide band beacons.

13. System (1) according to one of Claims 11 and 12, **characterized in that** the physiological sensors (7) arranged in the individual electronic case comprise a cardiofrequency meter whose signal is processed so as to obtain the heart rate of each individual.

14. System (1) according to any one of Claims 1 to 5, optimized to detect stress in an individual in a professional situation, **characterized in that** the motion sensors (5) arranged in the individual electronic case comprise an accelerometer and **in that** the physiological sensors (7) comprise a cardiofrequency meter and an electroencephalograph.

15. System (1) according to any one of Claims 1 to 5, optimized for advanced physiological tests on individuals, **characterized in that** the motion sensors (5) arranged in the individual electronic case comprise one or more sensors taken from a tri-axial accelerometer, a magnetometer, a pressure sensor, a gyroscope, and a position sensor of GPS type, an ultrasound sensor of Zigbee type or an ultra-wide band sensor, and **in that** the physiological sensors (7) comprise one or more sensors taken from an electromyograph, at least one temperature sensor, an electrocardiograph ECG, an electroencephalograph EEG, each of said sensors (5, 7) having at least one channel.
